# EUROPEAN PATENT SPECIFICATION

(11) **EP 4 271 210 B1**
(45) Date of publication and mention of the grant of the patent: **30.10.2024**
(21) Application number: 21848192.7
(22) Date of filing: 28.12.2021
(51) Int. Cl.: A23L 33/105, A61K 36/87, A61K 31/122, A61K 31/19, A61K 31/352, A61P 27/06, A61K 36/45

(54) **GRAPE SEED EXTRACT PREPARATION PROCESS AND EXTRACTS THUS OBTAINED**
VERFAHREN ZUR HERSTELLUNG VON TRAUBENKERNEXTRAKTEN UND DIE SO GEWONNENEN EXTRAKTE
PROCÉDÉ DE PRÉPARATION D'EXTRAITS DE PÉPINS DE RAISIN ET EXTRAITS AINSI OBTENUS

(30) Priority: 30.12.2020 IT 202000032765
(43) Date of publication of application: 08.11.2023
(73) Proprietor: Distillerie Bonollo Umberto - S.p.A. Con Sigla "U.B. S.p.A.", 35035 Mestrino (PD) (IT)
(72) Inventor: MORAZZONI, Paolo, 22100 Como (IT); URSINI, Fulvio, 35122 Padova (IT); SANTINELLO, Sandro, 35038 Torreglia (PD) (IT)
(74) Representative: Mittler, Andrea
(86) International application number: PCT/IB2021/062373
(87) International publication number: WO 2022/144762

(56) References cited:
- WO-A1-2005/036988
- CN-A- 106 045 959
- CN-A- 106 496 176
- CN-A- 108 095 118
- US-A- 5 484 594
- US-A1- 2007 071 871
- US-A1- 2018 303 895
- US-A1- 2020 390 726
- LORENZO RODRÍGUEZ J M ET AL: "ANTIMICROBIAL AND ANTIVIRAL ACTIVITIES OF GRAPE SEED EXTRACTS", 1 January 2016 (2016-01-01), XP055794947, Retrieved from the Internet <URL:https://www.novapublishers.com/wp-content/uploads/2018/10/978-1-63484-578-6_ch10.pdf> [retrieved on 20210413]

## Description

### FIELD OF THE INVENTION

The present invention relates to a process for preparing grape seed extracts from selected unfermented marc, as well as extracts thus obtained and the use thereof in nutraceutical and pharmaceutical compositions.

### PRIOR ART

Extracts obtained from grape seeds originating from different cultivars of *Vitis vinifera L.* and containing polyphenols of catechetical origin have been used for a long time both as drugs for treating chronic peripheral venous insufficiency and as supplements/food supplements (nutraceuticals), in particular relating to cardiovascular well-being. This last use has found great impetus since the early 1990s in relation to epidemiological observations. In fact, in this context, the low incidence of degenerative cardiovascular diseases compared to a high intake of dietary fats observed in the French population was for the first time associated with the concomitant consumption of polyphenols, in particular of oligomeric procyanidins deriving from catechin and epicatechin, originating from the processing of grapes and present in particular in red wine. Numerous literature data are now available to support the important role of oligomeric procyanidins in producing beneficial effects on the cardiovascular system and on visual function as well as indicating new applications thereof, e.g., related to viral infection control. CN 108 095 118 A discloses a slimming health food characterized in that it is prepared by mixing the following raw materials in parts by weight: grape seed extract dry powder 5-21, citrus peel dry powder 15-35, olive extract dry powder 5-17, and rouge radish extract dry powder 6-18, green pea dry powder 10 to 35, yam dry powder 10 to 28. The grape seed extract powder in CN 108 095 118 A is a product of edible grape seed extract produced by Shaanxi Zhongxin Biotech Co., Ltd. (the content of procyanidins is ≥ 95%).

Grape seed extracts normally used in both the pharmaceutical and nutraceutical fields and prepared according to the existing prior art contain oligomeric forms with a degree of polymerization between 2 and 10, polymeric forms and above all a consistent content, between 15 and 30%, of monomeric flavanols such as catechin and epicatechin. The latter products are considered undesirable and there are known cases of side effects which have led to the withdrawal of a pharmaceutical product containing catechin as the active ingredient, as the only component, from the market. Numerous attempts have also been made to selectively obtain certain oligomeric fractions by means of variable mixtures of solvents. For example, in US Patent 5,484,594 the monomer content has been reported not to exceed 1.5% of the extract, but the process involves a selective extraction of the monomeric components using mixtures of organic solvents such as acetone, methanol or other alcohols, therefore making these extracts unsuitable for nutraceutical use.

More recent attempts have also been made by means of the combined use of preliminary aqueous extraction and subsequent purification steps on adsorption chromatographic resins and elutions with hydroalcoholic mixtures, but even in this case no extracts with monomeric flavanol content lower than 10% have been obtained (WO 2016/020855 A1).

In CN106496176A, it is affirmed to have obtained powdered proanthocyanidins at purities of 99.5%. The described extraction method comprises the steps of pre-treating the grape seeds, by extraction with water and/or ultrasonic extraction to extract the pre-treated grape seeds to obtain an extract; filtering the extract with a microfiltration membrane, an ultrafiltration membrane and a nanofiltration membrane in sequence to obtain a filtrate; using a macromolecular resin to adsorb the filtrate, then using ethanol for the elution and collection of the eluate rich in procyanidins; Centrifugation, separation and concentration of the eluate rich in proanthocyanidins and concentration by three steps of reverse osmosis to obtain an extract and drying the extract using a freeze-drying method or a microwave method to obtain proanthocyanidin powder. However, it is not only observed that ethanol is used for the elution and collection of the eluate, but also that this method requires a rather long sequence of steps, which are also expensive as well as complex, making the method altogether uneconomical.

Thus, despite the existence of a certain number of procedures for the selective extraction of oligomeric procyanidins present in grape seeds, the industrial aspects of obtaining extracts with a low content of monomeric forms and which at the same time respect the native component of the biologically active oligomeric forms remain unsatisfied. In addition, the aspects of extractive procedural acceptability must still be considered unsatisfied, such as the solvents used and the level of contaminants which can make the same extracts usable in the relevant product fields, with particular reference to the nutraceutical fields.

Therefore, it is an object of the present invention to overcome the limitations and disadvantages of the known extraction processes.

### SUMMARY OF THE INVENTION

Said object has been achieved by a process for preparing grape seed extracts, as reported in claim 1.

In another aspect, the present invention relates to a grape seed extract obtainable by such a process, as reported in claim 4.

In a further aspect, the present invention relates to a nutraceutical or pharmaceutical composition, comprising such a grape seed extract, and suitable excipients.

As will be evident from the following detailed description, an innovative extraction process has been developed by means of infusion technology coupled with tangential filtration with selective membranes, which allows obtaining extracts with a low content of monomeric flavanols (≤5%) and high content of oligomeric procyanidins (≥95%). The process, which includes the use of only water as an infusion and extraction solvent, also allows obtaining extracts with acceptable limits of contaminants in compliance with international regulations and usable in the reference product fields, in particular the nutraceutical field.

### BRIEF DESCRIPTION OF THE DRAWINGS

The features and advantages of the present invention will become apparent from the following detailed description, from the embodiments provided by way of illustrative and non-limiting examples, and from the accompanying drawings, in which:
- Figure 1 shows the HPLC-GPC profile of the grape seed extract obtained in Example 1, compared with that of a commercial extract with a high monomer titer ('RP'),
- Figure 2 shows the almost overlappable FTIR spectrum of the grape seed extract of 6 distinct repetitions of Example 1, confirming the reproducibility of the process, and
- Figures 3A and 3B, obtained by quantitative processing of the analysis data carried out by LC-Chip/ESI-QTOF-MS in negative ionic mode, show the comparisons of the contents of total procyanidins (Fig.3A) and monomeric catechins, total procyanidins, catechins and catechins esterified with gallic acid, respectively (Fig. 3B).

### DETAILED DESCRIPTION OF THE INVENTION

The invention therefore relates to a process for the preparation of a grape seed extract, said process comprising the steps of:
i. providing marc deriving from grape pressing, and subjecting said marc to mechanical sieving, to separate the grape seeds from stalks, skins and other residues resulting from the mechanical pruning of the vines,
ii. drying the grape seeds thus separated, down to a residual humidity of less than 8%,
iii. infusing the grape seeds in water at a temperature of at least 80°C for at least an hour, at a pH of 3.0-3.5,
iv. separating the aqueous solution obtained in step iii., from the grape seeds,
v. subjecting said aqueous solution to tangential microfiltration through 0.2 micron membranes, then collecting and preserving the permeate,
vi. subjecting said permeate to tangential ultrafiltration with a cut off of 300 kDalton, then collecting and preserving the retentate,
vii. subjecting said retentate to tangential nanofiltration, to eliminate residual water, thus obtaining a grape seed extract, and optionally
viii. concentrating and spray drying said extract, thus obtaining a powder.

In particular, in step i., firstly the marc to be started in the process of the invention is selected. Preferably, it is marc obtained from single cellars pre-selected for quality assurance (freshness, procyanidin titer) of northeastern Italy, which are transferred immediately after pressing at the production site (distillery) and selected based on the morphological aspect thereof. The marc is then subjected to mechanical sieving to eliminate stalks, skins and other residues resulting from the mechanical pruning. To improve efficiency, it is preferable to carry out this step by using two sieves having different porosity.

In step ii., the grape seeds are then subjected to drying, preferably at a temperature of 70-110°C, and subsequently to cooling, checking that the residual humidity is less than 8%.

Preferably, the output temperature thereof, preferably at a temperature of 30-60°C, and the procyanidin titer, preferably ≥5%, are also checked.

In step iii., the grape seeds are infused in water at a temperature of at least 80°C for at least an hour, at a pH of 3.0-3.5. This is the actual extraction step, which can preferably be carried out with a batch system, in particular an infuser with an internal stirring system. In preferred embodiments, the stirring is activated for one minute every 15 minutes.

As stated above, the process of the present invention includes the use of only water as an infusion and extraction solvent. Since water is the only solvent used in the process of the invention, this means that no other solvents or mixtures are therefore used in any step of the process.

Preferably, step iii. is carried out at ambient pressure and with a water temperature of about 90°C for a time of about 2 hours.

The desired pH is preferably achieved and maintained by adding phosphoric acid.

In order to improve the extraction yield, it is possible (but not strictly necessary) to carry out a treatment with hydrolytic enzymes (for example, pectinase, cellulase, amylopectinase), before step iii. and/or during step iii., using a recirculation with ultrasound.

Preferably, at the end of step iii., the solution obtained is discharged by filtering it on a grille with holes of about 2 mm and the infusion is repeated for another two hours. At the end, the solution of the second extract is discharged, filtering it on a grille with holes of about 2 mm.

In this sense, step iii. can also be configured with systems provided with a double infusion mixer, thus increasing the overall efficiency.

In step iv., the aqueous extraction solution of step iii. is separated from the grape seeds.

Preferably, such a solution is then left to cool to a temperature below 60°C.

In step v., said aqueous extraction solution is subjected to tangential microfiltration through 0.2 micron membranes, collecting and preserving the permeate.

In fact, the following are obtained with the tangential microfiltration:
- a retentate, i.e., the fraction which does not pass through the 0.2 micron pores, and
- a permeate, i.e., the fraction which passes through the 0.2 micron pores, which is sent to a collection tank.

Preferably, the retentate is subjected to a diafiltration process after washing with water at 90°C and again to tangential microfiltration. The further permeate thus obtained is joined with the previous permeate while the residual retentate is discarded. This increases the final yield and the overall efficiency of the process.

In preferred embodiments, the permeate of the tangential microfiltration is percolated on a stationary phase of pyrolyzed resins which removes over 95% of the pesticides possibly contained in the extract. The elution ratio is preferably 5 volumes per hour on 1 volume of stationary phase. The resins are then regenerated, for example, with a steam treatment at 120°C which removes the adsorbed elements, making the resins operational again. In these embodiments, the permeate is then cooled to below 55°C.

In step vi., said permeate is subjected to tangential ultrafiltration with a cut off of 300 kDalton, collecting and preserving the retentate.

Therefore, in this step the permeate, i.e., the fraction not retained by the membranes, is discarded, while the retentate thus obtained is stored in a lung and preferably partially passed through the tangential ultrafiltration. The discarded permeate is strongly enriched with monomeric flavanols, while the retentate is enriched with oligomeric procyanidins.

The monomeric flavanols can be recovered from the tangential ultrafiltration permeate using DA201E resin, i.e., a highly hydrophobic polystyrene/vinylbenzene-based resin, subsequently eluted with diluted soda solution and subsequent passage on cationic resin for neutralization. This solution can then possibly be used in the step following the tangential nanofiltration to give final products with variable monomeric flavanol (e.g., 40%) and procyanidin content (e.g., 60%).

Preferably, the retentate is subjected to a second ultrafiltration step (diafiltration) which increases the purity thereof and which requires the gradual addition of water at 90°C to maintain a constant volume during the washing step before the final concentration.

In step vii., said retentate is then subjected to tangential nanofiltration, to eliminate residual water, thus obtaining a grape seed extract.

In fact, the tangential nanofiltration also eliminates small molecules such as mineral salts. The water is discarded as a permeate, while the retentate forms the grape seed extract according to the present invention.

In preferred embodiments, the retentate of the tangential nanofiltration is sent to a vacuum concentrator, which operates at a temperature of 25°C. The concentrate thus obtained is cooled to 0-2°C, placed in a small tank and stored in a cold room.

Preferably, the retentate, even concentrated, is sent for drying with a spray-dryer system.

In preferred embodiments, said spray-dryer system is set at the following operating conditions:
- Inlet air temperature: 190°C
- Outlet temperature: 80°C
- Contact time with air: about 2 seconds
- Air volume by weight of retentate: about 300 m³/kg

The dry extract thus obtained is stable at room temperature.

Finally, the grape seed extract thus obtained, i.e., in the form of dry powder, follows a packaging process, preferably in a 1 kg format, with a polylaminate film barrier with aluminum.

Preferably, the process of the invention essentially consists of the steps i. to viii. described above. For the purposes of the present invention, the expression "essentially consists of" means that steps i. to viii. are the only determining steps for obtaining the grape seed extract of the invention, other possible steps being merely accessory and/or of final packaging.

In other preferred embodiments, the process of the invention consists of steps i. to viii. described above.

It should be understood that the preferred and advantageous aspects of the process of the invention as described above are similarly preferred and advantageous also for the embodiments of the process essentially consisting of steps i. to viii, as well as for the embodiments of the process consisting of steps i. to viii.

In another aspect, the present invention relates to a grape seed extract obtainable by such a process, said extract being according to claim 4. It follows that the grape seed extract obtainable by the process of the invention can comprise at most traces of water, being instead completely free of organic solvents.

The amount of monomeric flavanols is measured by HPLC (on a dry base).

The amount of oligomeric procyanidins is measured according to the Bate-Smith method.

The grape seed extract obtainable by the process of the invention is characterized by:
≥ 80% of total polyphenols, as catechin - Folin-Ciocalteu reagent
≥ 95% oligomeric procyanidins - Bate Smith
0.05-0.5% gallic acid - HPLC (on a dry base)
1.0-5.0% monomers (epicatechin+catechin) - HPLC (on a dry base)
1.0-5.0% dimers (Procyanidin B1 + Procyanidin B2) - HPLC (on a dry base)

As will be seen from the following Examples, the grape seed extract of the invention advantageously has high antioxidant capacities, further supporting the convenience of the preparation process described above. In fact, such high antioxidant capacities confirm that the process of the invention has not altered these properties in any way, but conversely has enhanced them.

In a further aspect, the present invention relates to a nutraceutical or pharmaceutical composition, comprising such a grape seed extract, and suitable excipients.

The term "Excipient" means a compound or respective mixture suitable for use in a nutraceutical or pharmaceutical formulation. For example, an excipient for use in a pharmaceutical formulation should not, in general, cause a side effect in a subject, nor should it significantly inhibit the efficacy of the active ingredients.

Suitable excipients are acidifying agents, acidity correctors, anti-caking agents, antioxidants, additives, resistance agents, gelling agents, glazing agents, modified starches, sequestering agents, thickeners, sweeteners, thinners, solvents, disintegrating agents, slip agents, dyes, binders, lubricants, stabilizers, absorbents, preservatives, wetting agents, flavors, film-forming substances, emulsifiers, wetting agents, release retardants and mixtures thereof.

Preferably, said excipients are starch, modified starch, cellulose, modified cellulose, microcrystalline cellulose, sodium carboxymethyl cellulose, pectin, tragacanth, mannitol, dicalcium phosphate, xanthan gum, carrageenan, sodium alginate, guar gum, maltodextrin, silicon dioxide, or mixtures thereof.

The composition of the present invention can be prepared by processes known in the art. In fact, for oral administration, the components can, for example, be mixed with one or more excipients, enclosed in the form of a soft gel capsule, tablet, mini-tablet, micro-tablet, granules, micro-granules, pellets, multi-particles, micronized particles, powder, solution, suspension, dispersion, emulsion, gel, drops or aerosol.

In some embodiments, the composition of the invention can further comprise at least one other plant extract, such as bilberry extract.

In preferred embodiments, the composition of the invention further comprises at least one active ingredient selected from quercetin, glycyrrhizic acid, astaxanthin, and mixtures thereof.

For the purposes of the present invention, the expression "further comprises" means that the composition is further added with an ingredient, regardless of the possible presence thereof in the grape seed extract.

The composition of the invention can be in unit dose form.

Preferably, said unit dose comprises 20-200 mg of grape seed extract of the invention.

Preferably, said unit dose comprises 20-200 mg of quercetin.

Preferably, said unit dose comprises 1-100 mg of glycyrrhizic acid.

Preferably, said unit dose comprises 0.1-20 mg of astaxanthin.

In preferred embodiments, even of the unit dose, the composition of the invention comprises the grape seed extract of the invention, quercetin, and glycyrrhizic acid. These embodiments find advantageous application in the treatment of viral infections.

The term "treatment" refers to the effects of the composition of the invention which is capable of imparting a benefit to patients, both human and animal, suffering from an infectious disease, for example an improvement in the patient's condition or a delay in the progression of the disease. Such a composition can also have a preventive, as well as curative effect, against infection. In the present document, the term "infection" or its synonym "infectious disease" means the invasion, colonization and/or multiplication of a microorganism within or on another host organism. The term "infection" refers to an infectious disease caused by a virus, in particular respiratory viruses. For the purposes of the present invention, a virus is preferably selected from respiratory syncytial virus, influenza virus, parainfluenza virus, metapneumovirus, rhinovirus, adenovirus, and coronavirus.

Both coronavirus and influenza virus are structurally characterized by a lipid bilayer-based casing (derived from the cell membrane systems of the host cell). The lipid bilayer also contains glycoproteins which can protrude outward and in many cases are responsible for the highly responsive recognition of the respective host cells, the associated physical interactions, subsequent adhesion and thus also the invasion of the virus into the host cell. Examples of these proteins are hemagglutinin from influenza viruses and S proteins from coronaviruses. The proven relevant entry point of such viruses through droplet infection is the nasopharyngeal area and thus the viruses differentiate in terms of subsequent colonization of epithelial host cells which for influenza virus is more likely the bronchial area, while for SARS-Cov-2 the nasopharyngeal area.

On this basis it seems conceivable to consider the protection of the nasal area, mouth and throat against the penetration of viral particles as an effective strategy against the COVID-19 pandemic. This approach should also be reinforced by the ability to control virus replication early in the infection.

In this context, it has recently been shown that a number of botanicals are endowed with interesting biological properties which seem indicated for the specific purpose of counteracting the first stages of infection and propagation of the virus through the nasopharyngeal area. In this sense, without wishing to be bound by any theory, the combination of grape seed procyanidins, quercetin and glycyrrhizic acid of the above preferred embodiments is believed to contribute to the inhibition of virus penetration into host cells, as well as the ability to inhibit viral replication in the initial step of infection in the nasopharyngeal area, representing a valid strategy for controlling infection.

The composition of these embodiments can be in the form of slow-release oral tablets or even nasal/oral sprays. The dosages can be calculated based on inhibitory *in vitro* concentrations reported for both penetration and viral replication and taking into account the area covered by the mucosa of the nasopharyngeal cavity, which does not exceed a volume of 100 mL.

In other preferred embodiments, even of the unit dose, the composition of the invention comprises the grape seed extract of the invention, and bilberry extract, astaxanthin, or both.

These embodiments find advantageous application in the treatment of glaucoma.

Glaucoma is currently one of the main causes of blindness in the world. Open angle glaucoma (OAG), the most common type of glaucoma, is characterized by slowly progressive remodeling of the optic nerve head (ONH) and loss of the optic nerve fiber layer (RNFL) in combination with changes in the optic nerve field of vision corresponding to increased excavation of the optic disc. Primary OAG has been divided into high pressure OAG (POAG) when intraocular pressure is > 21 mmHg and normal tension glaucoma (NTG) when intraocular pressure is within the normal range (<21 mmHg).

OAG is a multifactorial optic neuropathy of unknown etiology; elevated intraocular pressure is the most important risk factor for the disease, although the exact pathways linking ocular hypertension with glaucomatous optic neuropathy and associated visual field loss have not yet been elucidated. Intraocular pressure is also the most studied risk factor because it is clinically treatable and easily measurable. Mechanisms other than intraocular pressure, measured in NTG patients, can also contribute to glaucomatous damage.

There are two possible mechanisms postulated as causal of POAG:
- vascular dysfunction causing ischemia in ONH - Chronic impairment of the blood supply is also associated with other risk factors such as advanced age, systemic vascular disease, myopia. In addition to being one of the most relevant etiological causes, vascular dysfunction is also a concomitant phenomenon which contributes to the damage of neural tissues. As a result, the modulation of vascular dysfunction, particularly improving ocular blood flow, is becoming the second treatment option for glaucoma.
- Mechanical dysfunction causing compression of the axons.

A credible body of evidence is now supporting the idea that vascular dysfunctions, particularly common during the aging process, are mainly due to vascular structural changes and endothelial dysfunctions.

The latter is highly dependent on the balance in the production and release of NO and ET-1.

On this basis, without wishing to be bound to any theory, it is believed that some botanical derivatives such as anthocyanosides and procyanidins present in the above embodiments of the invention contribute to the reduction of endothelial dysfunctions and vascular structural changes, with consequent reduction of the impaired and elevated intraocular hemodynamic pressure.

In particular, anthocyanosides modulate the hyperpermeability of the ciliary capillaries while the procyanidins of the grape seed extract can simultaneously improve endothelial function and hemodynamics. These combined effects, converging in the normalization of the capillary filtration of the ciliary body and parallel to the action of astaxanthin, can act together in the reduction of intraocular pressure and related damage.

It should be understood that all the possible combinations of the preferred aspects of the steps of the process, of the extract, of the composition, and of the relative uses as indicated above are also described, and therefore similarly preferred.

It should also be understood that all the aspects identified as preferred and advantageous for the process and the extract are to be considered similarly preferred and advantageous also for the composition and the active compounds thereof, and the uses thereof.

Below are working examples of the present invention provided for illustrative purposes.

### EXAMPLES

### Example 1.

### Preparation of a grape seed extract according to the present invention

Marc obtained from single cellars pre-selected for quality assurance (freshness, procyanidin titer) of northeastern Italy is transferred immediately after pressing at the production site (distillery) and selected based on the morphological aspect thereof. The marc is then subjected to sieving by means of two sieves with pores of different diameters to eliminate stalks, skins and other residues resulting from the mechanical pruning. The grape seeds are then subjected to drying (70-110°C) and then to cooling with control of the outlet temperature thereof at 40°C, of the residual humidity (<8%) and of the procyanidin titer (≥5%).

### Process:

| | |
|---|---|
| Botanical variety | *Vitis Vinifera L.* |
| Parts used | Whole seeds |
| Extraction solvent | water |
| W/v extraction ratios | 1 : 20-25 |

The extraction is carried out with a batch system.

800 kg of selected dry grape seeds are taken and placed in an extraction mixer of about 5,000 liters and the first 3,200 liters of demineralized hot water at 90/95°C and acidified with phosphoric acid (up to pH 3.0- 3.5). The extractor is provided with a jacket which can be fed with hot water and an internal stirrer which can be activated in a programmed manner. In the current state, the mixing is activated for one minute every 15 minutes. In order to improve the extraction yield, it is also possible (but not essential) to introduce a step with hydrolytic enzymes (pectinase, cellulase, amylopectinase) before the start of the extraction process and/or to use a recirculation with ultrasound system during the actual extraction process.

At the end of the two hours of extraction, the solution obtained is discharged by filtering it on a grille with porosity of approximately 2 mm and a second extraction is carried out with 3,000 liters of demineralized water at 90°C acidified with phosphoric acid (keeping the pH at 3.0-3.5), for another two hours and at the end the solution of the second extract is discharged by filtering it on a grille with holes of about 2 mm.

The extraction process described above can also be configured with systems provided with a double infusion mixer, increasing the efficiency of the process itself.

The extracted seeds are discarded, with the elimination of about 500 liters of water, while the two extraction solutions, after cooling below 60°C, are sent to a tangential microfiltration system through 0.2 micron membranes, from here two fractions are obtained:
- the retentate, i.e., that which does not pass through the 0.2 micron pores, about 200 liters, which is subjected to a diafiltration process after washing with 400 liters of demineralized water at 90°C (subjected again to microfiltration) and the further permeate obtained is joined with the previous permeate, while the retentate residue is discarded.
- The permeate, i.e., that which passes through the 0.2 micron pores, about 5,900 liters, is sent to a collection tank.

The permeate of the tangential microfiltration is sent on a stationary phase of pyrolyzed resins which removes more than 95% of contaminants, including pesticides, possibly contained in the extract, the elution ratio is 5 volumes per hour on 1 volume of resins. The resins are then regenerated with a steam treatment at 120°C which removes the contaminants adsorbed on the resins, making them operational again.

The solution treated on the pyrolyzed resin is then cooled below 55°C and sent to a tangential ultrafiltration system with a cut off of 300 kDalton. The permeate (that which passes through the membranes) is discarded while the retentate thus obtained is stored in a lung and partially passed through the tangential ultrafiltration again.

The ultrafiltration permeate, rich in monomeric flavanols, can be recovered by means of DA201E resin subsequently eluted with diluted soda solution and subsequent passage on cationic resin for neutralization. This solution can then possibly be used in the following nanofiltration step to give rise to final products with variable content of monomeric flavanols (e.g., 40%) and procyanidins (e.g., 60%).

At the end of this ultrafiltration step approximately 1,100 liters of retentate are obtained which is subjected to a second ultrafiltration step (diafiltration) which increases the purity thereof and which requires the gradual addition of 2,000 L of hot water at 90 °C to the tank as the ultrafiltration concentrates the retentate and to keep it always at a constant volume, the operation continues until it has discarded 2,000 liters of water.

The ultrafiltration retentate is then subjected to a nanofiltration which removes water and small molecules such as mineral salts and further increases the purity of the polyphenols by reducing the amount of water. The water is discarded as a permeate, while the product as a retentate, about 300 liters, passes to the next step.

The retentate of the nanofiltration is sent to the vacuum concentrator which operates at a temperature of 25°C. The concentrator produces about 90-100 liters of concentrate. The concentrate is cooled to 0-2°C, placed in a small tank and stored in a cold room.

The concentrated liquid product is sent for drying with a spray-dryer system which operates under the following operating conditions:
- Inlet air temperature: 190° C
- Outlet temperature: 80° C
- Air product contact time: about 2 seconds
- Air volume by product weight: about 300 m³/kg

Finally, a packaging process in 1 kg format follows, with a polylaminate film barrier with aluminum. From this last step, about 30-35 kg of dry grape seed extract are obtained, of an orange-light brown color, with the expected polyphenolic profile and reported in table 1.

**Table 1.**

| Components | Methods | Result |
|---|---|---|
| Total polyphenols (as catechins) (procyanidins) | Folin-Ciocalteu reagent (accuracy ± 15%) | ≥ 80% |
| Oligomeric procyanidins | Bate Smith | ≥ 95% |
| Gallic acid | HPLC (on a dry base) | 0.05-0.5% |
| Monomers (epicatechin+catechin) | HPLC (on a dry base) | 1.0-5.0% |
| Dimers (Pro B1 + Pro B2) | HPLC (on a dry base) | 1.0-5.0% |

The product thus obtained is stable at room temperature.

### Characterization of the extract:

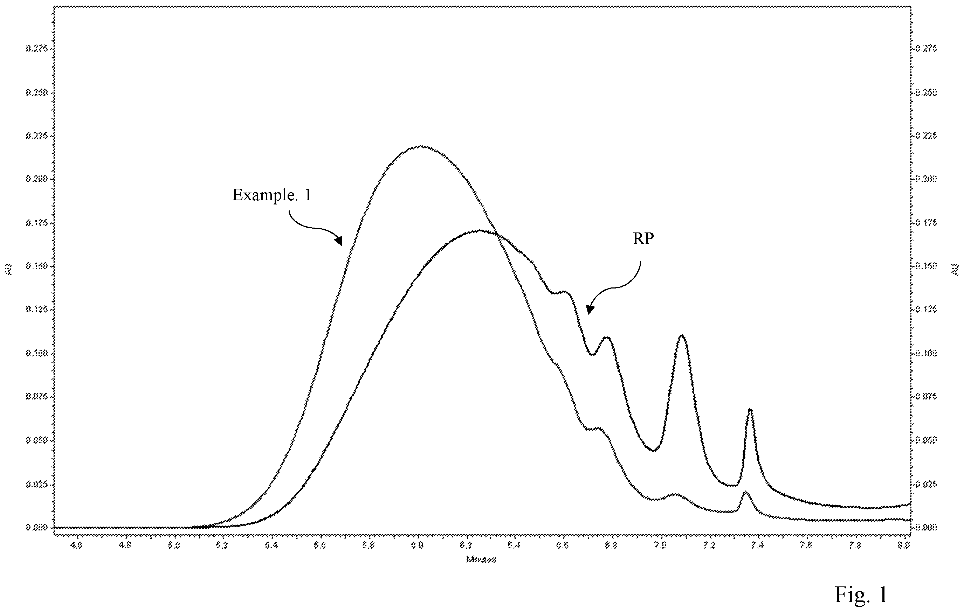

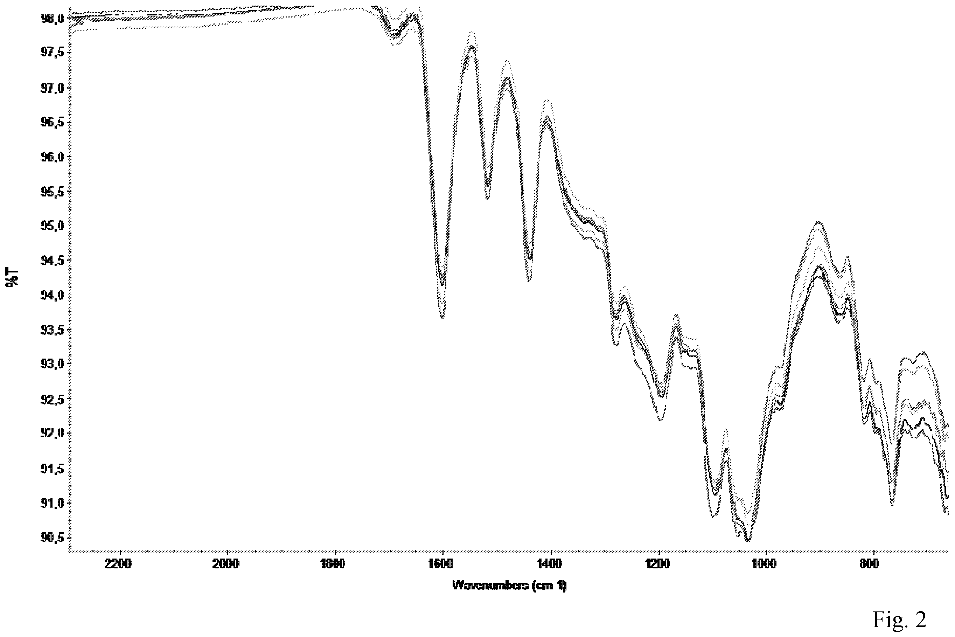

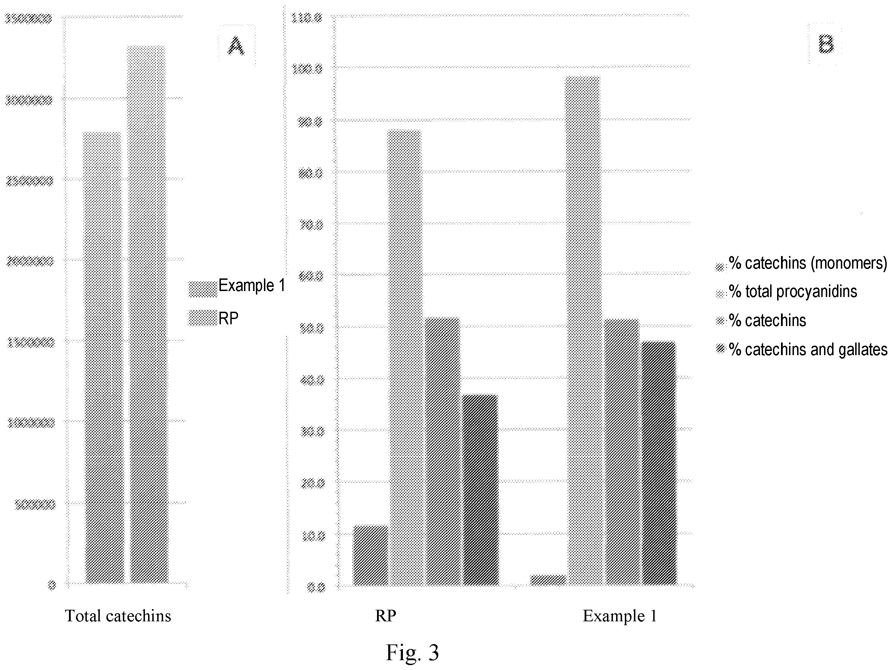

The extract obtained as above was characterized by the following analytical techniques: HPLC-GPC (Figure 1), FTIR (Figure 2) and mass spectrometry (Figure 3).

The outcome of such analyses was compared with a reference commercial grape seed extract (briefly 'RP'), comprising 5-15% monomers, and ≥85% oligomeric procyanidins.

As could be observed, in particular from Figure 3, the extract obtained from Example 1 showed a percentage of procyanidins of about 99%, while the percentage of monomeric catechins was about 1%.

### Example 2.

### Evaluation of the antioxidant capacity

### DPPH test

The DPPH test allows determining the antioxidant power by reacting the sample to be analyzed with a solution of DPPH [2,2-diphenyl-1-picrylhydrazyl, PM 394.33, C18H72N506] and analyzing the decrease in the peak at 517 of the DPPH* radical at UV. Antioxidant compounds which are capable of transferring a hydrogen atom to the radical cause a loss of absorbance of the solution. The decrease of the DPPH* radical peak at 517 nm is then analyzed by spectrophotometry after a predetermined incubation time. This decrease (discoloration) is proportional to the antioxidant load present in the sample. The results can also be expressed as IC₅₀, i.e., the concentration of antioxidant compound which causes an initial 50% decrease in DPPH'.

Three samples obtained according to the process described in Example 1 were tested and the relative results reported in the following table:

| | DPPH IC₅₀ (µg/ml) |
|---|---|
| Extract Example 1 (batch 1) | 2.097±0.136 |
| Extract Example 1 (batch 2) | 3.219±0.422 |
| Extract Example 1 (batch 3) | 3.324±0.238 |
| RP | 2.012±0.324 |
| Ascorbic acid | 7.196±0.852 |

### ORAC test

The ORAC method (Oxygen Radical Absorbance Capacity) is one of the most common methods for determining radical trapping capacity against peroxyl radicals. The principle of the test is based on the measurement of the decrease in the fluorescence intensity of a fluorescent target molecule (for example fluorescein), under a constant flow of peroxyl radicals, generated by the thermal decomposition of an azo compound. The results are expressed as equivalent Trolox^{®} micromoles, using this compound as the reference standard.

Three samples obtained according to the process described in Example 1 were tested and the relative results reported in the following table:

| | ORAC (µmοl Trolox^{®} equivalent/g of sample |
|---|---|
| Extract Example 1 (batch 1) | 15210±2356 |
| Extract Example 1 (batch 2) | 14834±1902 |
| Extract Example 1 (batch 3) | 15128±2150 |
| RP | 16340±2150 |
| Ascorbic acid | 2900±310 |

### Example 3.

The following composition was prepared, in unit dose form, comprising the following active ingredients:

| | |
|---|---|
| Extract Example 1 | 50 mg |
| Quercetin | 50 mg |
| Glycyrrhizic acid | 20 mg |

and suitable excipients.

This composition can be used in the treatment of viral diseases, in particular of the respiratory tract.

### Example 4.

The following composition was prepared, in unit dose form, comprising the following active ingredients:

| | |
|---|---|
| Extract Example 1 | 60 mg |
| Bilberry extract | 100 mg |
| Astaxanthin | 6 mg |

and suitable excipients.

This composition can be used in the treatment of eye diseases, in particular glaucoma.

### Example 5

The following composition was prepared, in unit dose form, comprising the following active ingredients:

| | |
|---|---|
| Extract Example 1 | 60 mg |
| Astaxanthin | 6 mg |

and suitable excipients.

This composition can be used in the treatment of eye diseases, in particular glaucoma.

## Claims

1. Process for the preparation of a grape seed extract, said process comprising the steps of:
1. providing marc deriving from grape pressing, and subjecting said marc to mechanical sieving, to separate the grape seeds from stalks, skins and other residues resulting from the mechanical pruning of the vines,
2. drying the grape seeds thus separated, down to a residual humidity of less than 8%,
3. infusing the grape seeds in water at a temperature of at least 80°C for at least an hour, at a pH of 3 .0-3.5,
4. separating the aqueous solution obtained in step 3, from the grape seeds,
5. subjecting said aqueous solution to tangential microfiltration through 0.2 micron membranes, then collecting and storing the permeate,
6. subjecting said permeate to tangential ultrafiltration with a cut off of 300 kDalton, then collecting and preserving the retentate,
7. subjecting said retentate to tangential nanofiltration, to eliminate residual water, thus obtaining a grape seed extract, and optionally
8. concentrating and spray drying said extract, thus obtaining a powder.

2. The process of claim 1, wherein before step 3 and/or during step 3, a treatment with hydrolytic enzymes, such as pectinase, cellulase, or amylopectinase, is carried out.

3. The process of claim 1 or 2, wherein the permeate of the tangential microfiltration of step 5 is sent to a stationary phase of pyrolyzed resins, to remove contaminants possibly present in the extract.

4. A grape seed extract obtainable by the process of any one of claims 1-3, said extract being free of organic solvents and comprising ≤ 5 wt% monomeric flavanols and ≥ 95 wt% oligomeric procyanidins, based on the weight of the extract, wherein the extract is **characterized by**:
≥ 80% of total polyphenols, as catechin (method: by Folin-Ciocalteu reagent; accuracy ± 15),
≥ 95% oligomeric procyanidins (method: Bate Smith),
0.05-0.5% gallic acid (method: HPLC on a dry base,
1.0-5.0% monomers (epicatechin+catechin) (method: HPLC on a dry base), and
1.0-5.0% dimers (Procyanidin B1 + Procyanidin B2) (method: HPLC on a dry base).

5. Nutraceutical or pharmaceutical composition, comprising the grape seed extract of claim 4, and suitable excipients.

6. The composition of claim 5, further comprising quercetin and glycyrrhizic acid.

7. The composition of claim 6 for use in the treatment of viral infections.

8. The composition of claim 5, further comprising bilberry extract, astaxanthin, or both.

9. The composition of claim 8, for use in the treatment of glaucoma.

## Patentansprüche

1. Verfahren zur Herstellung eines Traubenkernextrakts, wobei das Verfahren die folgenden Schritte umfasst:
1. Bereitstellen von Trester, das aus der Traubenpressung stammt, und mechanisches Sieben dieses Tresters, um die Traubenkerne von den Stielen, Schalen und anderen Rückständen zu trennen, die beim mechanischen Beschneiden der Rebstöcke anfallen,
2. Trocknen der so abgetrennten Traubenkerne bis zu einer Restfeuchtigkeit von weniger als 8 %,
3. Einlegen der Traubenkerne in Wasser bei einer Temperatur von mindestens 80°C für mindestens eine Stunde bei einem pH-Wert von 3,0-3,5,
4. Trennung der in Schritt 3 erhaltenen wässrigen Lösung von den Traubenkernen,
5. Durchführen einer tangentialen Mikrofiltration der wässrigen Lösung durch 0,2-Mikrometer-Membranen und anschließendes Sammeln und Lagern des Permeats,
6. Durchführen einer tangentialen Ultrafiltration des Permeats mit einer Trenngrenze von 300 kDalton und anschließendes Sammeln und Aufbewahren des Retentats,
7. Durchführen einer tangentialen Nanofiltration mit dem Retentat, um Restwasser zu entfernen und so einen Traubenkernextrakt zu erhalten, und gegebenenfalls
8. Konzentrieren und Sprühtrocknen des Extrakts, um ein Pulver zu erhalten.

2. Verfahren nach Anspruch 1, wobei vor Schritt 3 und/oder während Schritt 3 eine Behandlung mit hydrolytischen Enzymen, wie Pektinase, Cellulase oder Amylopektinase, durchgeführt wird.

3. Verfahren nach Anspruch 1 oder 2, wobei das Permeat der tangentialen Mikrofiltration von Schritt 5 in eine stationäre Phase aus pyrolysierten Harzen geleitet wird, um möglicherweise im Extrakt vorhandene Verunreinigungen zu entfernen.

4. Traubenkernextrakt, erhältlich durch das Verfahren nach einem der Ansprüche 1 bis 3, wobei der Extrakt frei ist von organischen Lösungsmitteln und enthält: ≤5 Gew.-% monomere Flavanole und ≥95 Gew.-% oligomere Procyanidine, bezogen auf das Gewicht des Extrakts, wobei der Extrakt **gekennzeichnet ist durch**:
≥ 80 % der gesamten Polyphenole, wie Catechin (Methode: **durch** Folin-Ciocalteu-Reagenz; Genauigkeit ± 15),
≥ 95% oligomere Procyanidine (Methode: Bate Smith),
0,05-0,5% Gallussäure (Methode: HPLC auf trockener Basis,
1,0-5,0% Monomere (Epicatechin+Catechin) (Methode: HPLC auf trockener Basis), und
1,0-5,0% Dimere (Procyanidin B1 + Procyanidin B2) (Methode: HPLC auf trockener Basis).

5. Nutrazeutische oder pharmazeutische Zusammensetzung, die den Traubenkernextrakt nach Anspruch 4 und geeignete Hilfsstoffe enthält.

6. Zusammensetzung nach Anspruch 5, die ferner Quercetin und Glycyrrhizinsäure enthält.

7. Zusammensetzung nach Anspruch 6 zur Verwendung bei der Behandlung von viralen Infektionen.

8. Zusammensetzung nach Anspruch 5, die ferner Heidelbeerextrakt, Astaxanthin oder beides enthält.

9. Zusammensetzung nach Anspruch 8 zur Verwendung bei der Behandlung des Glaukoms.

## Revendications

1. Procédé pour la préparation d'un extrait de pépins de raisin, ledit procédé comprenant les étapes de :
1. fourniture de marc dérivant du pressage du raisin, et soumission dudit marc à un tamisage mécanique pour séparer les pépins des rafles, peaux et autres résidus résultant de la taille mécanique des vignes,
2. séchage des pépins de raisin ainsi séparés, jusqu'à une humidité résiduelle inférieure à 8 %,
3. infusion des pépins de raisin dans de l'eau à une température d'au moins 80°C pendant au moins une heure, à un pH de 3,0 à 3,5,
4. séparation de la solution aqueuse obtenue dans l'étape 3 d'avec les pépins de raisin,
5. soumission de ladite solution aqueuse à une microfiltration tangentielle à travers des membranes de 0,2 micromètre, puis collecte et stockage du perméat,
6. soumission dudit perméat à une ultrafiltration tangentielle avec une séparation à 300 kilodaltons, puis collecte et conservation du rétentat,
7. soumission dudit rétentat à une nanofiltration tangentielle, pour éliminer l'eau résiduelle, ce qui donne ainsi un extrait de pépins de raisin, et éventuellement
8. concentration et séchage par pulvérisation dudit extrait, ce qui donne ainsi une poudre.

2. Procédé selon la revendication 1, dans lequel, avant l'étape 3 et/ou pendant l'étape 3, un traitement avec des enzymes hydrolytiques, telles qu'une pectinase, une cellulase, ou une amylopectinase, est effectué.

3. Procédé selon la revendication 1 ou 2, dans lequel le perméat de la microfiltration tangentielle de l'étape 5 est envoyé à une phase stationnaire de résines pyrolysées, pour éliminer les contaminants éventuellement présents dans l'extrait.

4. Extrait de pépins de raisin pouvant être obtenu par le procédé de l'une quelconque des revendications 1 à 3, ledit extrait étant exempt de solvants organiques et comprenant ≤ 5 % en poids de flavanols monomères et ≥ 95 % en poids de procyanidines oligomères, par rapport au poids de l'extrait, lequel extrait est **caractérisé par** :
≥ 80 % de polyphénols totaux, sous forme de catéchine (procédé : par le réactif de Folin-Ciocalteu, précision ± 15),
≥ 95 % de procyanidines oligomères (procédé : Bate Smith),
0,05 à 0,5 % d'acide gallique (procédé : HPLC sur une base sèche),
1,0 à 5,0 % de monomères (épicatéchine + catéchine) (procédé : HPLC sur une base sèche), et
1,0 à 5,0 % de dimères (procyanidine B1 + procyanidine B2) (procédé : HPLC sur une base sèche).

5. Composition nutraceutique ou pharmaceutique comprenant l'extrait de pépins de raisin selon la revendication 4, et des excipients convenables.

6. Composition selon la revendication 5, comprenant en outre de la quercétine et de l'acide glycyrrhizique.

7. Composition selon la revendication 6, pour une utilisation dans le traitement d'infections virales.

8. Composition selon la revendication 5, comprenant en outre de l'extrait de myrtille, de l'astaxanthine, ou les deux.

9. Composition selon la revendication 8, pour une utilisation dans le traitement d'un glaucome.
